# EUROPEAN PATENT APPLICATION

(11) **EP 4 631 566 A1**
(43) Date of publication of application: **15.10.2025**
(21) Application number: 24168930.6
(22) Date of filing: 08.04.2024
(51) Int. Cl.: A61N 1/39

(54) **INTEGRATED RESISTANCE MONITORING FOR A DEFIBRILLATOR ELECTRODE PLATE**

(71) Applicant: Koninklijke Philips N.V., 5656 AG Eindhoven (NL)
(72) Inventor: WU, Jikun, 5656 AG Eindhoven (NL); YANG, Chao, 5656 AG Eindhoven (NL); CHEN, Chaobin, 5656 AG Eindhoven (NL)
(74) Representative: Philips Intellectual Property & Standards

(57) **Abstract**

A processing system for measuring the resistances of an electrode plate of a defibrillator paddle. The processing system is formed as part of a defibrillator comprising the defibrillator paddle and a paddle tray configured to receive the defibrillator paddle. The processing system is configured to measure at least one first side resistance of a first side of the electrode plate using a plurality of first detection points in electrical contact with the first side of the electrode plate. The processing system is also configured to measure at least one second side resistance of a second side of the electrode plate using a plurality of second detection points, formed as part of the paddle tray, that come in electrical contact with the second side of the electrode plate when the defibrillator paddle is received by the paddle tray.

## Description

### FIELD OF THE INVENTION

The present invention relates to the field of defibrillators, and in particular to systems and methods for monitoring the resistance of an electrode plate of a defibrillator paddle.

### BACKGROUND OF THE INVENTION

Defibrillators play an important role in the life-saving treatment of patients suffering from cardiac arrest and heart arrythmia. Able to provide an electric `shock' to a patient's heart, defibrillators rely on good electrical conductivity of the electrodes of the defibrillator for transferring the electric shock to the patient.

In hospitals, a conductive gel may be applied to the electrodes to aid in the defibrillation process. However, repeated exposure of the electrodes to the gel, and the leakage of the gel into the internal circuitry of the electrodes, can result in the electrical performance of the electrodes reducing over time, due to effects such as oxidation and rust of the electrode metal material. Furthermore, the electrode performance can additionally be affected by deformation of the electrodes (e.g., caused from improper use or poor upkeep of the electrodes) and the general deterioration of the electrodes from repeated use.

Eventually, this can result in the electrodes being unable to provide a sufficient electric shock to the patient and/or being unsafe to use, e.g., due to the potential danger of an electrical fault or spark.

Therefore, it is desirable for defibrillators to have a mechanism that can automatically detect whether the electrodes are suitable to use.

### SUMMARY OF THE INVENTION

The invention is defined by the claims.

According to examples in accordance with an aspect of the invention, there is provided a processing system configured to measure the resistances of an electrode plate of a defibrillator paddle. The processing system is configured to measure at least one first side resistance of a first side of the electrode plate using a plurality of first detection points in electrical contact with the first side of the electrical plate, and measure at least one second side resistance of a second side of the electrode plate using a plurality of second detection points in electrical contact with the second side of the electrical plate, in which the plurality of second detection points is formed as part of a paddle tray configured to receive the defibrillator paddle.

The present disclosure provides a technique for measuring resistances of an electrode plate of a defibrillator paddle. According to the present disclosure, a plurality of first detection points, in electrical contact with the first side of the electrode plate, is used to determine first side resistances of the electrode plate. Furthermore, a plurality of second detection points, formed in a paddle tray, is used to determine second side resistances of the electrode plate. More particularly, when the defibrillator paddle is received by (i.e. stored in) the paddle tray, the plurality of second detection points are configured to come into electrical contact with the second side of the electrode plate.

It is known that the electrical properties/performance of an electrode plate of a defibrillator paddle (e.g., electrode plate conductance, defibrillation efficiency/effectiveness) may deteriorate over time due to effects such as rust, oxidation, and deformation, which can negatively affect the performance of the defibrillator paddle. Thus, it is desirable to monitor the electrical properties of an electrode plate to determine when the electrode plate is unfit for purpose.

According to the above context, each possible pair of detection points functions as a probe to assess the electrical properties of the electrode plate. More specifically, a pair of detection points may be chosen for which the resistance of the electrode plate is measured between, e.g., by passing a small current through the electrode plate (i.e. from one detection point to another detection point) and measuring the potential difference between the pair of detection points.

It has been recognized that, by using both first detection points (which are in electrical contact with the first side of the electrode plate) and second detection points (which come into electrical contact with the second side of the electrode plate), the resistance of both the first side and the second side of the electrode plate can be measured. In particular, the resistance of both sides of the electrode plate can be measured without needing to mount specific measuring devices on both sides of the electric plate.

The processing system is configured to measure at least one (e.g., depending on the number of first detection points) first side resistance of the electrode plate between any two first detection points of the plurality of first detection points and at least one (e.g., depending on the number of second detection points) second side resistance of the electrode plate between any two second detection points of the plurality of second detection points.

The present invention therefore permits the resistance of the electrode plate, on both the first side and the second side, and potentially across different parts of the electrode plate (depending on the number and position of detection points) to be monitored. More particularly, both first side and second side resistances can be monitored without the need to directly (e.g., and permanently) mount or connect monitoring elements to both sides of the electric plate.

In some embodiments, the processing system may be configured to iteratively measure the at least one first side resistance at a first time interval and measure the at least one second side resistance at a second time interval. This approach may be useful for employment when the defibrillator paddle is left in a "standby" mode, i.e., wherein the defibrillator paddle is receiving power but is not in use. Preferably, when in standby mode, each defibrillator paddle is received by a respective paddle tray, such that both the first side resistance(s) and the second side resistance(s) of the electrode plate may be measured.

According to the above approach, the resistance of the electrode plate may be measured at regular time intervals, e.g., every hour after first receiving power, to maintain a consistent monitoring of the condition of the electrode plate.

In some embodiments, the processing system may be configured to compare each first side resistance and each second side resistance to a respective threshold and, in response to any first side resistance and/or any second side resistance breaching the respective threshold, generate an alert indicator. A respective threshold may be chosen that, if breached, indicates the electrode plate as being unsafe and/or unfit for use.

Furthermore, generating an alert indicator may comprise generating, at a user interface communicatively coupled to the processing system, a user-perceptible output. Accordingly, a user will be alerted, by the user-perceptible output, to the potential risk posed by the (current) electrode plate.

Also provided is a defibrillator comprising the processing system, the defibrillator paddle, and the paddle tray configured to receive the defibrillator paddle. The defibrillator paddle comprises the electrode plate for applying an electric shock to a subject, comprising the first side and the second side, and the plurality of first detection points in electrical contact with the first side of the electrode plate. Additionally, the paddle tray comprises the plurality of second detection points configured to, when the defibrillator paddle is received by the paddle tray, come into electrical contact with the second side of the electrode plate.

This embodiment provides a defibrillator comprising at least one defibrillator paddle for applying (when handled by a user) an electric shock to a subject, e.g., a patient suffering cardiac arrest. More specifically, each defibrillator paddle comprises an electrode plate which (when in use) is pressed against the subject to provide the electric shock, e.g., through the discharge of an electrical current/electrical charge from the electrode plate. The electrode plate comprises a first side and a second side, wherein the second side comes into contact with the subject when in use.

The defibrillator further comprises at least one paddle tray configured to receive a respective defibrillator paddle. Each paddle tray functions as a storage container for the respective defibrillator paddle when not in use. When received by the paddle tray, the second side of the electrode plate is in contact with the paddle tray.

In some embodiments, the paddle tray may comprise a receiving element configured to receive the defibrillator paddle at a first side of the paddle tray. Additionally, each of the plurality of second detection points may extend, from a second side of the paddle tray, opposite to the first side of the paddle tray, through a respective hole in the receiving element to the first side of the paddle tray.

In this way, the second side of the electrode plate may only come into contact with the plurality of second detection points and not the remainder of the processing system and/or any mounting elements of the second detection points. In other words, the receiving element of the paddle tray forms a barrier between the electrode plate and the remainer of the processing system.

In some embodiments, each of the plurality of second detection points may be a resilient protrusion. In this way, when a respective defibrillator paddle is received by the paddle tray, each of the plurality of second detection points is effectively biased against the second side of the electrode plate, ensuring good electrical contact between each of the plurality of second detection points and the second side of the electrode plate.

Additionally, or alternatively, each of the plurality of first detection points may be biased, by a respective biasing member, against the first side of the electrode plate. In this way, good electrical contact is maintained between each of the plurality of first detection points and the first side of the electrode plate.

In some embodiments, the defibrillator may further comprise an electrical shock system configured to, responsive to an activation indicator and only when the electrical shock system is turned on, provide an electric shock using the defibrillator paddle. Additionally, the processing system may be configured to measure the at least one first side resistance and the at least one of second side resistance, respectively, when the electrical shock system is turned on.

Using this approach, the resistance (and therefore condition) of the electrode plate is checked each time before use, i.e., before the electric shock is provided to the subject. A user may therefore confirm a defibrillator paddle (or more specifically the electrode plate of a defibrillator paddle) is in good condition before use, i.e., from the measured resistances.

In some embodiments, the number of first detection points may be no less than 4, and/or the number of second detection points may be no less than 4.

Additionally, or alternatively, the number of first detection points may be different from the number of second detection points.

Additionally, or alternatively, each of the plurality of first detection points may be no less than 2 cm away from any other of the plurality of first detection points, and each of the plurality of second detection points may be no less than 2 cm away from any other of the plurality of second detection points.

In some embodiments, each of the plurality of first detection points may electrically contact a respective first contact point of the first side of the electrode plate, and each of the plurality of second detection points may come into electrical contact with a respective second contact point of the second side of the electrode plate, when the defibrillator paddle is received by the paddle tray.

In some embodiments, each of the plurality of first detection points may be arranged to contact a periphery of the first side of the electrode plate, and each of the plurality of second detection points may be arranged to come into contact with a periphery of the second side of the electrode plate when the defibrillator paddle is received by the paddle tray.

In this way, the resistance of the electrode plate may be monitored over a greater area of the first side and the second side. Put another way, in contrast, if the detection points were arranged to come into contact only with central points of the first side or second side of the electrode plate, then the processing system would be unable to measure the resistance of peripheral regions of the first side or second side of the electrode plate.

Also provided is a method for measuring resistances of an electrode plate of a defibrillator paddle. The method comprises measuring at least one first side resistance of a first side of the electrode plate using a plurality of first detection points in electrical contact with the first side of the electrical plate, and measuring at least one second side resistance of a second side of the electrode plate using a plurality of second detection points in electrical contact with the second side of the electrical plate, wherein the plurality of second detection points is formed as part of a paddle tray configured to receive the defibrillator paddle.

In some embodiments, the method may further comprise comparing each first side resistance and each second side resistance to a respective threshold and, in response to any first side resistance and/or any second side resistance breaching the respective threshold, generating an alert indicator.

Furthermore, generating an alert indicator may comprise generating, at a user interface, a user-perceptible output.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 provides a processing system according to an embodiment of the invention;
Fig. 2 provides a schematic of a defibrillator according to an embodiment of the invention;
Fig. 3 provides an exploded view of a defibrillator paddle of the defibrillator;
Fig. 4 provides an exploded view of a paddle tray of the defibrillator;
Fig. 5 illustrates a first detection board according to an example;
Fig. 6 illustrates a second detection board according to an example; and

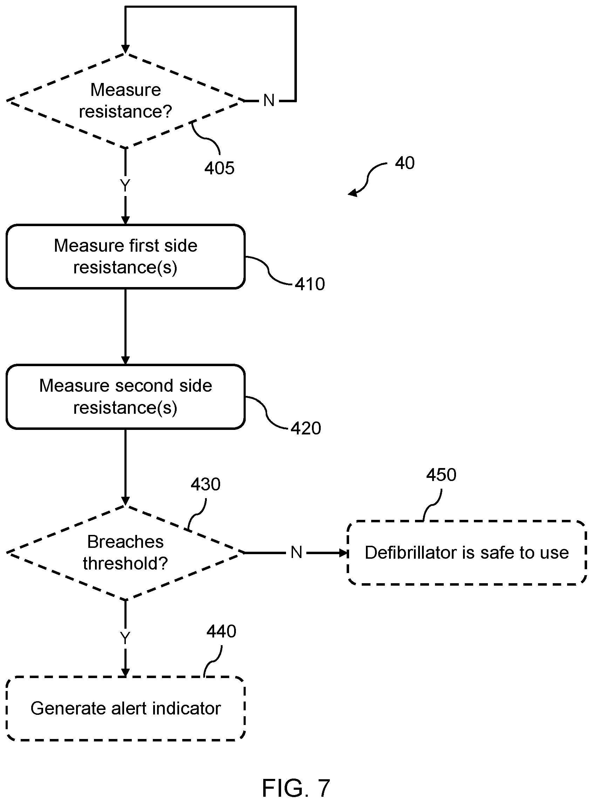

Fig. 7 provides a flowchart illustrating a proposed method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a processing system for measuring the resistance(s) of an electrode plate of a defibrillator paddle. The processing system uses a plurality of first detection points, that are in electrical contact with a first side of the electrode plate, to measure one or more resistances. In particular, the processing system is configured to measure one or more resistances of the first side of the electrode plate using the plurality of first detection points, i.e., measure the resistances between any pairs of first detection points.

Additionally, the processing system uses a plurality of second detection points formed as part of a paddle tray. The paddle tray is configured to receive the defibrillator paddle such that the plurality of second detection points come into electrical contact with a second side of the electrode plate. Furthermore, when the defibrillator paddle is received by the paddle tray, the processing system is configured to measure one or more resistances of the second side of the electrode plate using the plurality of second detection points, i.e., measure the resistances between any pairs of second detection points.

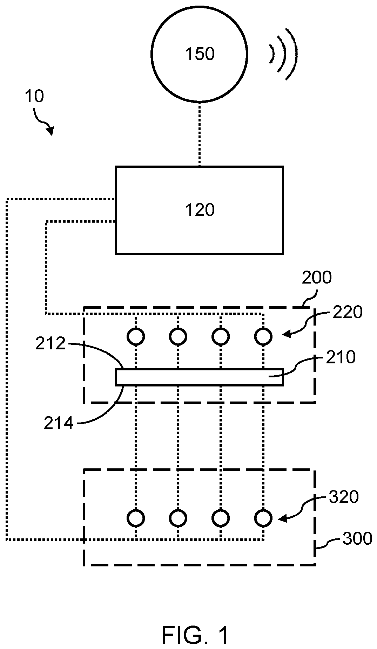

Fig. 1 shows an example of a system 10 according to the present disclosure, which may form (part of) a defibrillator. The system comprises a processing system 120, a plurality of first detection points 220 and a plurality of second detection points 320.

The plurality of first detection points 220 are formed as part of a defibrillator paddle 200 further comprising an electrode plate 210. The electrode plate 210 comprises a first side 212 and a second side 214, opposite to the first side 212. In some examples, the electrode plate 210 may have a substantially flat shape such that the first side 212 and the second side 214 correspond with the largest (i.e., in area) sides/faces of the electrode plate 210, e.g., a top and bottom face.

The defibrillator paddle 200 may be used as part of a defibrillator for the purpose of providing an electric shock to a subject. Specifically, the defibrillator paddle 200 functions as an electrode for transferring the electric shock from the defibrillator to the subject. In particular, the electric shock is transferred to the subject through the electrode plate 210, which is held in contact with the subject when providing the electric shock.

Each of the plurality of first detection points 220 makes electrical contact with the first side 212 of the electrode plate 210. Put another way, each of the plurality of first detection points 220 electrically contacts a (different) point on the first side 212 of the electrode plate 210, thereby forming an array of (electrical) contact points between the plurality of first detection points 220 and the first side 212 of the electrode plate 210.

Each of the plurality of first detection points 220 may therefore relay electrical signals (e.g., an electrical current and/or voltage) to/from the first side 212 of the electrode plate 210. In this way, a pair of first detection points (i.e., two of the plurality of first detection points 220) may function as a probe to measure the electrical properties of the first side 212 of the electrode plate 210.

Specifically, when passing a (known) current between a pair of first detection points, through the electrode plate 210, a potential difference between the pair of first detection points can be measured. It is therefore possible to estimate the resistance of the first side 212 of the electrode plate 210, i.e., a first side resistance, by dividing the measured potential difference by the current. By utilizing the plurality of first detection points 220, several first side resistances may be measured, i.e., through different pair combinations of the plurality of first detection points 220, a plurality of first side resistances between any two first detection points 220 may be measured.

The plurality of second detection points 320 are formed as part of a paddle tray 300. The paddle tray 300 is configured to receive the defibrillator paddle 200, e.g., so as to function as a receptacle for the defibrillator paddle 200, or otherwise engage with the defibrillator paddle 200. The paddle tray 300 is further configured such that, when the defibrillator paddle 200 is received by the paddle tray 300, the plurality of second detection points 320 come into electrical contact with the second side 214 of the electrode plate 210.

Put another way, when the defibrillator paddle 200 is received by the paddle tray 300, each of the plurality of second detection points 320 electrically contacts a (different) point on the second side 214 of the electrode plate 210, thereby forming an array of (electrical) contact points between the plurality of second detection points 320 and the second side 214 of the electrode plate 210.

Each of the plurality of second detection points 320 may therefore relay electrical signals (e.g., an electrical current and/or voltage) to/from the second side 214 of the electrode plate 210. In this way, a pair of second detection points (i.e., two of the plurality of second detection points 320) may function as a probe to measure the electrical properties of the second side 214 of the electrode plate 210.

Specifically, when passing a (known) current between a pair of second detection points, through the electrode plate 210, a potential difference between the pair of second detection points can be measured. An estimate of the resistance of the second side 214 of the electrode plate 210, i.e., a second side resistance, can thus be computed from dividing the measured potential difference by the current. By utilizing the plurality of second detection points 320, several second side resistances may be measured, i.e., through different pair combinations of the plurality of second detection points 320, a plurality of second side resistances between any two second detection points 320 may be measured.

Each detection point of the plurality of first detection points 220 and the plurality of second detection points 320 is communicatively coupled to the processing system 120. For example, each detection point may be electrically coupled to the processing system 120, e.g., via a physical electrical connection, such that each detection point may communicate with the processing system 120 (and vice versa) through electrical signals. Alternatively, each detection point may be wirelessly coupled with the processing system 120 and communicate, e.g., via a wireless transmitter for sending and/or receiving wireless signals.

The processing system 120 may send signal(s) to the plurality of (first and/or second) detection points for the purposes of measuring at least one first side resistance and/or at least one second side resistance of the electrode plate 210. Additionally, the processing system 120 may receive signals from the plurality of detection points that are at least indicative of the at least one first side resistance and/or the at least one second side resistance of the electrode plate 210. More specifically, the processing system 120 may use the received signal(s) to compute the at least one first side resistance and/or the at least one second side resistance.

For example, when electrically coupled to each detection point, the processing system 120 may be configured to pass an electrical current (e.g., from the processing system 120) through the first side 212 and/or second side 214 of the electrode plate 210 between a pair of first detection points and/or a pair of second detection points. Simultaneously, the processing system 120 may be configured to send an electrical signal to the pair of first detection points and/or the pair of second detection points indicative of measuring a potential difference between the pair of first detection points and/or the pair of second detection points. The processing system 120 may then receive electrical signals from the plurality of detection points indicating the measured potential difference(s) (e.g., analogue signals) which the processing system then uses to compute at least one first side resistance and/or at least one second side resistance of the electrode plate 210.

In this way, the processing system 120 is able to determine the resistance of the electrode plate 210, on both the first side 212 and the second side 214, without the need for additional external measuring devices/elements to be mounted onto the electrode plate 210. Additionally, by using a plurality of detection points for both the first side 212 and the second side 214, the resistance of the electrode plate 210 can be measured across different regions of the first side 212 and the second side 214, thereby providing information on the spatial variation of the resistance of the first side 212 and the second side 214 of the electrode plate 210.

In some examples, the processing system 120 may be configured to iteratively measure the at least one first side resistance and the at least one second side resistance at regular time intervals. More specifically, the processing system may measure the at least one first side resistance at a first time interval and measure the at least one second side resistance at a second time interval. Each time interval refers to an amount of time that has passed after a previous first or second side resistance measurement, e.g., 10 minutes, 1 hour, 24 hours, etc. Therefore, the time period between each consecutive first or second side resistance measurement may be the same. In this way, the resistance of the electrode plate 210 can be regularly and consistently monitored.

After measuring at least one first side resistance and at least one second side resistance, the processing system 120 may compare each first side resistance and each second side resistance to a respective threshold. For example, the processing system 120 may determine if each first side resistance and each second side resistance breach the respective threshold.

Each first side resistance and each second side resistance may be compared to a different respective threshold, i.e., each resistance measurement is compared to a unique resistance threshold. Alternatively, each first side resistance may be compared to a first respective threshold, e.g., a first side threshold, and each second side resistance may be compared to a second respective threshold, e.g., a second side threshold. Finally, each first side resistance and each second side resistance may instead be compared to the same respective threshold. For example, the threshold can be set as 15.5Ω.

The respective threshold may represent a maximum or minimum resistance of the electrode plate 210 (or a region of the electrode plate 210) for which the electrode plate 210 is considered safe to use. Accordingly, the processing system 120 may generate an alert indicator in response to any first side resistance and/or any second side resistance breaching the respective threshold. The alert indicator may at least indicate to a user that the electrode plate 210 is considered unsafe/unfit for use, thereby informing the user to not use the defibrillator paddle 200 and/or to replace the electrode plate 210.

In some examples, the processing system 120 may be communicatively coupled to a user interface 150. The user interface 150 may comprise any number of visual and/or audio elements (e.g., displays, lights, speakers, etc.) for providing information to a user in the form of a user-perceptible visual and/or audio output.

Accordingly, as part of generating an alert indicator, the processing system 120 may generate (at the user interface 150) a user-perceptible output, e.g., a visual and/or audio output. The user-perceptible output may be highly noticeable, e.g., a flashing light and/or loud sound, so as to clearly notify the user of the respective threshold being breached and to signify to the user of the potential danger posed by the electrode plate 210. Hence, the user may take the proper actions afterwards, e.g., change a new defibrillator paddle.

The processing system 120, as described above, may be integrated into a defibrillator for the purposes of monitoring the resistance (and therefore condition) of an electrode plate of at least one defibrillator paddle of the defibrillator.

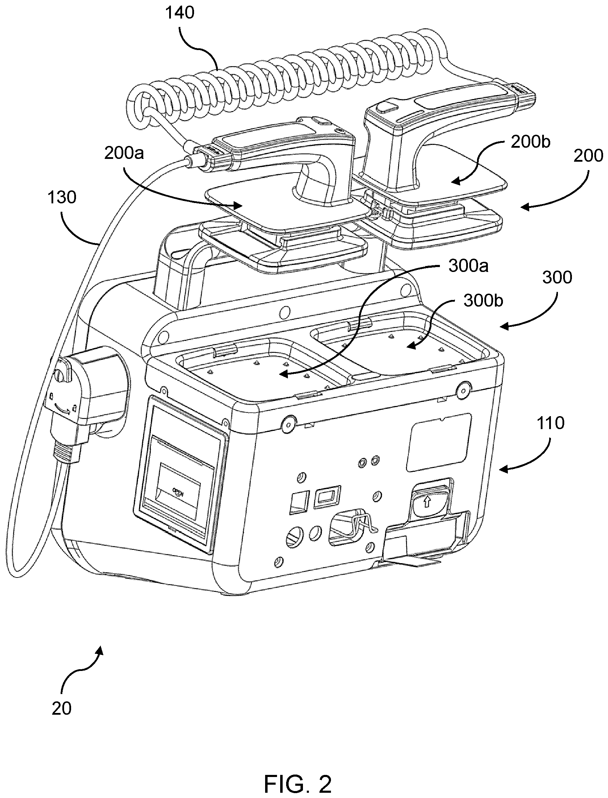

Fig. 2 shows a schematic of an example defibrillator 20 according to an embodiment of the invention. Specifically, the defibrillator 20 is a manual external defibrillator. However, it will be apparent to those skilled in the art how the present disclosure may apply to other types of defibrillator devices, such as automated external defibrillators, or advanced life support defibrillators.

The defibrillator 20 comprises at least one defibrillator paddle 200 and at least one paddle tray 300. More specifically, in the present example, the defibrillator 20 comprises a first defibrillator paddle 200a, a second defibrillator paddle 200b, a first paddle tray 300a and a second paddle tray 300b.

The first defibrillator paddle 200a and the second defibrillator paddle 200b are configured to work in conjunction for the purpose of providing an electric shock to a subject, such as a patient suffering from heart arrythmia and/or cardiac arrest. More specifically, when in use, the first defibrillator paddle 200a and the second defibrillator paddle 200b are placed/held against the subject's thorax and an electrical current and/or voltage (provided by a power source) is passed through the subject between the first defibrillator paddle 200a and the second defibrillator paddle 200b, thereby providing an electric shock to the subject.

The defibrillator 20 may further comprise a controller 110 electrically coupled to the first defibrillator paddle 200a and the second defibrillator paddle 200b. The controller 110 may at least function as a power source for providing the electrical power/current/voltage required for the electric shock. More precisely, the controller 110 may comprise an electrical shock system (not visible in Fig. 2) for providing the electric shock to the subject via the first defibrillator paddle 200a and the second defibrillator paddle 200b.

When the electrical shock system is turned on, i.e., receives electrical power from a mains supply or internal battery, the electrical shock system stores an amount of electrical energy, e.g., in a capacitor. In response to an activation indicator, e.g., a user input provided to the electrical shock system, the electrical shock system releases the stored electrical energy in the form of the electric shock, which is provided to the defibrillator paddles.

The controller 110 may further comprise a user interface (not visible in Fig. 2), such as the user interface 150 of Fig. 1. The user interface may permit a user to control aspects of the defibrillator 20, such as the amount of electrical energy stored by the electrical shock system, in addition to allowing the user to provide the activation indicator to the electrical shock system. The user interface may also provide information to the user in the form of a user-perceptible output, e.g., a visual and/or audio message or alert.

In the present example, the controller 110 is electrically coupled to the first defibrillator paddle 200a via a first flexible cable 130, passing between the controller 110 and the first defibrillator paddle 200a. The controller 110 is further electrically coupled to the second defibrillator paddle 200b via both the first flexible cable 130 and a second flexible cable 140, passing between the first defibrillator paddle 200a and the second defibrillator paddle 200b. The controller 110 is therefore permitted to (electrically) communicate (via the first flexible cable 130 and the second flexible cable 140) with both defibrillator paddles for the purposes of providing the electric shock. Additionally, the first flexible cable 130 and the second flexible cable 140 allow the defibrillator paddles to be easily maneuvered relative to the controller 110, e.g., when positioning the defibrillator paddles on a subject.

In some examples, however, each defibrillator paddle 200 may be electrically coupled individually to the controller 110, i.e., each defibrillator paddle 200 is not directly connected to another defibrillator paddle. In another example, each defibrillator paddle 200 may not be connected to a controller, i.e., the defibrillator does not comprise a controller. This may be permitted, e.g., when each defibrillator paddle 200 comprises its own power source, such as an internal battery.

According to the present example, the first paddle tray 300a is configured to receive the first defibrillator paddle 200a, e.g., for the purposes of safely and/or securely storing the first defibrillator paddle 200a when the defibrillator 20 is not in use. Similarly, the second paddle tray 300b is configured to receive the second defibrillator paddle 200b. However, it will be clear to those skilled in the art how each paddle tray 300 may be configured to receive a different defibrillator paddle than that described above. Alternatively, each paddle tray 300 may be configured to receive any defibrillator paddle, i.e., that there is no required restriction as to which paddle tray a specific defibrillator paddle may be received by/in.

Furthermore, it will be apparent how the defibrillator 20 of the present disclosure may comprise a number of defibrillator paddles and/or paddle trays different from the present example. For example, the defibrillator 20 may comprise only a single paddle tray 300 configured to receive both a first defibrillator paddle and a second defibrillator paddle. In another example, the defibrillator 20 may only comprise a single defibrillator paddle 200, e.g., and provide the electric shock in conjunction with an electrode from another device. Alternatively, the defibrillator 20 may only comprise a single defibrillator paddle 200 configured according to the present disclosure, i.e., and comprise a further defibrillator paddle/electrode configured differently from the present disclosure.

In the present example, the first paddle tray 300a and the second paddle tray 300b are depicted as being arranged on the controller 110. However, in other examples, one or more of the at least one paddle tray 300 may be arranged separately from the controller 110.

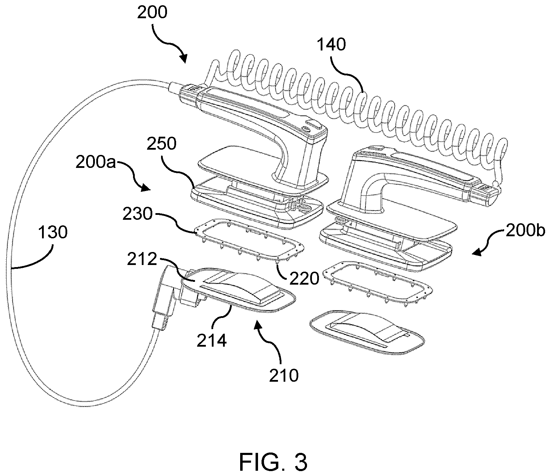

Fig. 3 shows an exploded view schematic of the first defibrillator paddle 200a and the second defibrillator paddle 200b of Fig. 2.

Each defibrillator paddle 200 comprises an electrode plate 210 for providing an electric shock to a subject. More specifically, the electric shock is produced by a power source (e.g., the controller 110 and/or electric shock system of Fig. 2) and transferred to the subject via the electrode plate 210 when the electrode plate 210 is held in contact with the subject. In particular, the electrode plate comprises a first side 212 and a second side 214, in which the second side 214 comes into contact with the subject when providing the electric shock.

Each defibrillator paddle 200 also comprises a plurality of first detection points 220 in electrical contact with the first side 212 of the electrode plate 210. When used as part of a processing system (e.g., the processing system 120 of Fig. 1) the plurality of first detection points 220 can be used to measure at least one first side resistance of the electrode plate 210.

In the present example, the plurality of first detection points 220 are arranged on a first detection board 230. The first detection board 230 may function as a supportive frame for the plurality of first detection points 220. Furthermore, the first detection board 230 may comprise a printed circuit board for which the plurality of first detection points 220 are soldered onto and coupled, via circuitry of the printed circuit board and/or electrical components on the printed circuit board, to other elements of the defibrillator and/or processing system (e.g., the processor 120 of Fig. 1).

In combination, the plurality of first detection points 220 and the first detection board 230 may constitute an add-on unit for an existing defibrillator paddle. More specifically, when provided as a single element, the plurality of second detection points 320 and the second detection board 330 may be integrated into an existing defibrillator paddle for providing the functionality of measuring the resistance of the first side of the electrode plate.

In some embodiments, each of the plurality of first detection points 220 may be biased against the first side 212 of the electrode plate 210. For example, each first detection point may be coupled to the first detection board 230 via a respective biasing member that biases each first detection point against the first side 212. Examples of suitable biasing members include springs, elastic plugs, resilient wedges and so on. Use of biasing members helps maintain a good electrical contact between each of the plurality of first detection points 220 and the first side 212, even when the electrode plate 210 becomes compressed and/or deformed, e.g., when being held against a subject.

Each defibrillator paddle may also comprise a paddle body 250. In the present example, the paddle body 250 functions as a protective housing for the other components of the defibrillator paddle 200, and also as a handle to allow a user to safely maneuver the defibrillator paddle 200. When the defibrillator paddle 200 is assembled, the first side 212 of the electrode plate 210 faces the paddle body 250 (while the second side 214 faces way from the paddle body 250) and is arranged such that the electrode plate 210 forms a closed volume with the paddle body 250. Furthermore, the plurality of first detection points 220 and the first detection board 230 are arranged within the (closed) volume bound by the electrode plate 210 and the paddle body 250.

It will be appreciated, however, how the present disclosure may apply to defibrillator paddles that do not comprise a paddle body, such as adhesive (defibrillator) electrode pads.

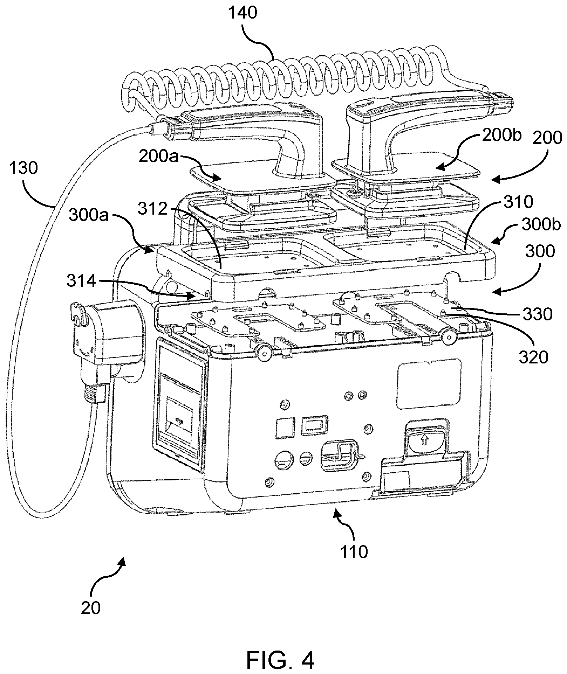

Fig. 4 shows another schematic view of the defibrillator 20 in addition to an exploded view of the first paddle tray 300a and the second paddle tray 300b.

Each paddle tray 300 may comprise a receiving element 310 for receiving a defibrillator paddle 200. More specifically, the receiving element 310 may be configured such that the defibrillator paddle 200 is received at a first side 312 of the paddle tray 300. The receiving element 310 may comprise a recess, receptacle, or slot whose shape is configured to accept the defibrillator paddle 200.

Each paddle tray 300 also comprises a plurality of second detection points 320. Each paddle tray 300 is configured such that, when a defibrillator paddle 200 is received by the paddle tray 300, the plurality of second detection points 320 come into electrical contact with the second side of the electrode plate of the defibrillator paddle 200. When used as part of a processing system (e.g., the processing system 120 of Fig. 1) the plurality of second detection points 220 can be used to measure at least one second side resistance of the electrode plate.

In the present example, the plurality of second detection points 320 are arranged on a second detection board 330. The second detection board 330 may function as a supportive frame for the plurality of second detection points 320. Furthermore, the second detection board 330 may comprise a printed circuit board for which the plurality of second detection points 320 are soldered onto and coupled, via circuitry of the printed circuit board and/or electrical components on the printed circuit board, to other elements of the defibrillator and/or processing system (e.g., the processing arrangement 120 of Fig. 1).

In combination, the plurality of second detection points 320 and the second detection board 330 may constitute an add-on unit for an existing paddle tray. More specifically, when provided as a single element, the plurality of second detection points 320 and the second detection board 330 may be integrated into an existing paddle tray for providing the functionality of measuring the resistance of the second side of the electrode plate of an existing defibrillator paddle.

According to the present example, the second detection board 330 may be arranged at a second side 314 of the paddle tray 300, opposite the first side 312 of the paddle tray 300. In this way, when the defibrillator paddle 200 is received by the paddle tray 300 (i.e., at the first side 312) the receiving element 310 forms a physical barrier between the defibrillator paddle 200 (or more specifically the electrode plate of the defibrillator paddle 200) and the second detection board 330. This may help to prevent the electrode plate of the defibrillator paddle 300 from touching electrical circuitry and/or electrical components on the second detection board 330.

Additionally, when the paddle tray 300 is assembled, each of the plurality of second detection points 320 may be configured to extend from the second side 314 of the paddle tray 300 through a respective hole in the receiving element 310 to the first side 312 of the paddle tray 300. In this way, though the receiving element 310 prevents the electrode plate from touching the second detection board 330, the plurality of second detection points 320 can still electrically contact the second side of the electrode plate.

In some embodiments, each of the plurality of second detection points 320 may be a resilient protrusion, e.g., each second detection point may comprise a spring or a compressive element, or itself be compressive and/or elastic, such that each of the plurality of second detection points 320 can be biased against the second side of the electrode plate (when received by the paddle tray 300). This ensures each of the plurality of second detection points 320 maintains good electrical contact with the second side of the electrode plate, but also allows each of the plurality of second detection points 320 to retract/be compressed towards the second side 314 of the paddle tray 300. In this way, when received by the paddle tray 300, the defibrillator paddle 200 can lie flush against the receiving element 310, i.e., as the plurality of second detection points 320 can be compressed into the holes of the receiving element 310 to form a flat surface. This results in a more uniform distribution of forces applied to the electrode plate when received by the paddle tray 300, preventing deformation of the electrode plate.

As mentioned previously, the controller 110 may comprise an electrical shock system and a user interface. The controller 110 may also comprise a processing system (not visible in Fig. 4), such as the processing system 120 illustrated in Fig. 1. The processing system, in conjunction with the plurality of first detection points and the plurality of second detection points 320, may form a system (e.g., the processing system 120 of Fig. 1) for measuring the resistance(s) of the electrode plate of the defibrillator paddle 200, i.e., the at least one first side resistance and/or the at least one second side resistance of the electrode plate.

In some embodiments, the processing system may be configured to measure the at least one first side resistance and the at least one second side resistance of the electrode plate of at least one of the defibrillator paddles 200 (i.e., the first defibrillator paddle 200a and/or the second defibrillator paddle 200b) when the electrical shock system is turned on, i.e., when the electrical shock system receives electrical power.

It is assumed that, in most cases, the electric shock system will only be turned on when the electric shock is to (shortly) be provided to a subject. Therefore, in the present embodiment, the resistances of the electrode plate may be checked (just) before the electric shock is provided to the subject. Accordingly, an alert indicator may be generated in response to any of the at least one first side resistance(s) and/or any of the at least one second side resistance(s) breaching a respective threshold, thereby alerting a user to a potentially defective electrode plate of at least one of the defibrillator paddles 200. The alert indicator may also be indicative of which defibrillator paddle 200 comprises the defective electrode plate.

Similar to the processing system 120 of Fig. 1, while the electrical shock system remains on, the processing system may continue to monitor the resistance(s) of the electrode plate by iteratively measuring the at least one first side resistance at a first time interval (i.e., a period of time after previously measuring the at least one first side resistance) and the at least one second side resistance at a second time interval (i.e., a period of time after previously measuring the at least one second side resistance). This may effectively represent a 'standby' mode of the defibrillator, in which elements of the defibrillator (e.g., the electrical shock system) are receiving power but are not in direct use. Accordingly, while in standby mode, each defibrillator paddle 200 may preferably be received by a respective paddle tray 300 so that the at least one second side resistances of the electrode plate(s) can be measured.

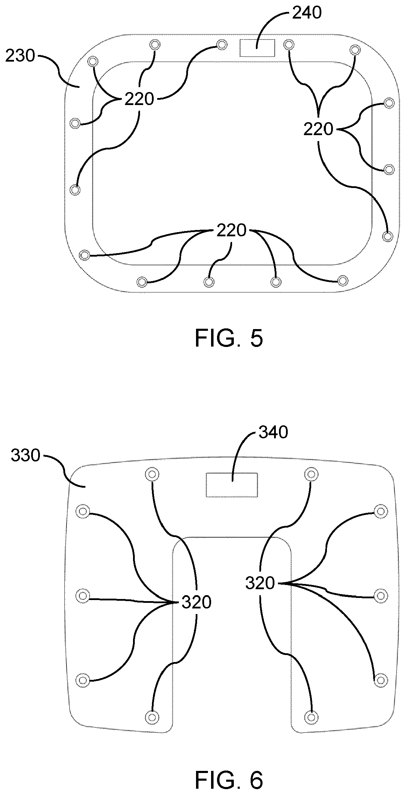

Figs. 5 and 6 schematically show examples of the first detection board 230 and the second detection board 330, respectively. The plurality of first detection points 220 and the plurality of second detection points 320 are arranged on the first detection board 230 and the second detection board 330, respectively. In one example, the plurality of first detection points 220 is arranged evenly on the periphery of the first detection board 230. In one example, the plurality of second detection points 320 is arranged evenly on the periphery of the second detection board 330.

Each detection board may comprise a processing module (or simply, processor). Specifically, a first processor 240 may be formed on the first detection board 230 and a second processor 340 may be formed on the second detection board 330. This may be the case when a controller of a defibrillator, comprising the first detection board 230 and the second detection board 330, does not comprise a processor.

The first processor 240 and the second processing 340 may together form a part of the processing system.

Accordingly, in the present examples, the first processor 240 is electrically coupled to each of the plurality of first detection points 220 and the second processor 340 is electrically coupled to each of the plurality of second detection points 320, thereby forming a processing system similar to the processing system 120 of Fig. 1 (except that the processing system 120 now comprises the first processor 240 and the second processor 340). As such, the first processor 240 may send/receive electrical signals to/from each of the plurality of first detection points 220 for the purposes of measuring at least one first side resistance of an electrode plate when the plurality of first detection points 220 are in electrical contact with a first side of the electrode plate. Similarly, the second processor 340 may send/receive electrical signals to/from each of the plurality of second detection points 320 for the purposes of measuring at least one second side resistance of an electrode plate when the plurality of second detection points 320 are in electrical contact with a second side of the electrode plate.

In some embodiments, such as the present example, the number of first detection points may be different to the number of second detection points. Explicitly, in Fig. 5, 15 first detection points 220 are arranged on the first detection board 230, whereas, in Fig. 6, 10 second detection points 320 are arranged on the second detection board 330.

A possible (e.g., maximum) number of first detection points and second detection points may be dependent on a specific configuration of the defibrillator paddle and the paddle tray, i.e., the amount of space provided by the defibrillator paddle and the paddle tray for accommodating first detection points and second detection points, respectively. Accordingly, this may result in the number of first detection points being different to the number of second detection points.

Further to the above, in some embodiments, each detection point may be no less than 2 cm away from any other detection point. Put another way, a circular area of radius 2 cm centered on each detection point may not contain any other detection point. There may therefore be a limitation as to how many detection points may be arranged within a given area.

Additionally, when brought into contact with a side of the electrode plate, each detection point may correspond to a respective contact point on the surface of the electrode plate, i.e., different from the contact point of any other detection point with the electrode plate surface. Explicitly, each of the plurality of first detection points 220 may electrically contact a respective first contact point of the first side of the electrode plate and each of the plurality of second detection points 320 may electrically contact a respective second contact point of the second side of the electrode plate.

A resistance measurement between a pair of detection points therefore corresponds to the resistance of the electrode plate between the contact points of the two detection points with the electrode plate.

In some embodiments, each detection point may be arranged to contact a periphery of the electrode plate, i.e., a position on or close to the edge of the electrode plate (on either the first side or the second side of the electrode plate). Specifically, each of the plurality of first detection points 220 may be arranged to contact a periphery of the first side of the electrode plate, and each of the plurality of second detection points 320 may be arranged to come into contact with a periphery of the second side of the electrode plate when the defibrillator paddle is received by the paddle tray.

In combination, the above features permit the plurality of first detection points 220 and the plurality of second detection points 320 (as part of the processing system) to measure the resistance(s) of the first side and the second side of the electrode plate, respectively, across different regions of the first side and the second side of the electrode plate, i.e., by utilizing different pair combinations of first or second detection points. This may allow defects in the electrode plate (e.g., rust, deformation, etc.) that are localized to specific regions of the first or second side of the electrode plate to be more easily identified, i.e., from a difference in the measured resistance of that region compared to surrounding regions.

Fig. 6 shows a flowchart illustrating an example method 40 to be implemented by the processing system for measuring (and assessing) the resistances of an electrode plate of a defibrillator paddle.

The method 40 may comprise a step 405 of determining whether or not to measure the resistances of the electrode plate. A positive determination in step 405 may be responsive to one or more predetermined conditions being satisfied. For example, when the processing system is part of a defibrillator that comprises an electrical shock system, step 405 may have a positive determination in response to the electrical shock system being turned on. Additionally, after an initial positive determination, step 405 may have a positive determination in response to a set amount of time passing after the initial positive determination, corresponding to measuring the resistances of the electrode plate at set time intervals.

In response to a negative determination in step 405, the method 40 may repeat step 405, i.e., the method 40 continues to determine/monitor whether or not to measure the resistances of the electrode plate until a positive determination is triggered. After a negative determination in step 405, the method 40 may wait a period of time before repeating step 405, i.e., there is a time delay between consecutive instances of step 405. This may be desired when implemented in a processing system to reduce on computing resources.

Following a positive determination in step 405, the method 40 proceeds to steps 410 and 420 for measuring at least one first side resistance of a first side of the electrode plate and at least one second side resistance of a second side of the electrode plate, respectively. Explicitly, the at least one first side resistance is measured between a plurality of first detection points in electrical contact with the first side of the electrode plate. For example, the resistances between any pairs of first detection points are measured. Additionally, the at least one second side resistance is measured between a plurality of second detection points that come in electrical contact with the second side of the electrode plate when the defibrillator paddle is received by a paddle tray that comprises the plurality of second detection points. For example, the resistances between any pairs of second detection points are measured.

Consequently, the at least one second side resistances may only be measured when the defibrillator paddle is received by the paddle tray. The method 40 may therefore skip step 420 if the defibrillator paddle is not being received by the paddle tray.

Following steps 410 and 420, the method 40 may proceed to a step 430 of comparing each first side resistance and each second side resistance to a respective threshold voltage. Specifically, step 430 comprises determining if each first side resistance and each second side resistance breaches (e.g., is higher or lower than) the respective threshold. In one example, the first side resistances between any two of the first detection points and the second side resistances between any two of the second detection points will be compared to the respective threshold. Referring to Fig. 5, the first side resistance between any two of the first detection points 220 will be measured and the measured first side resistances will be compared to the threshold respectively. The second side resistances between any two of the second detection points 320 will be measured and the measured second side resistances will be compared to the threshold respectively.

The respective threshold may represent a maximum or minimum resistance of the electrode plate above or below which the electrode plate is considered unsuitable for use, i.e., unsuitable for providing an electric shock to a subject due to the electrode plate being ineffective or unsafe. Accordingly, in response to a positive determination in step 430 (i.e., a first side resistance and/or a second side resistance breaching the respective threshold), the method 40 may proceed to a step 440 for generating an alert indicator.

The alert indicator may at least indicate to a user that the electrode plate is considered unsafe/unfit for use, thereby informing the user to not use the defibrillator paddle and/or to replace the electrode plate. Step 440 may comprise, as part of generating the alert indicator, generating a user-perceptible output at a user interface. The user-perceptible output may comprise a visual and/or audio output from the user interface, so as to clearly alert the user of the respective threshold being breached and to signify to the user of the potential danger posed by the electrode plate.

Alternatively, in response to a negative determination in step 430 (i.e., none of the first side resistances and second side resistances breaching the respective threshold), the method 40 may proceed to a step 450 for indicating that the defibrillator paddle is safe/suitable to use. Step 450 may comprise generating a further user-perceptible output (i.e., a user-perceptible output different to the user-perceptible output of step 440) at the user interface, e.g., a green light to indicate to the user it is safe to proceed with using the defibrillator paddle.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A processing system configured to measure the resistances of an electrode plate of a defibrillator paddle, the processing system being configured to:
measure at least one first side resistance of a first side of the electrode plate using a plurality of first detection points in electrical contact with the first side of the electrical plate; and
measure at least one second side resistance of a second side of the electrode plate using a plurality of second detection points in electrical contact with the second side of the electrical plate, wherein the plurality of second detection points is formed as part of a paddle tray configured to receive the defibrillator paddle.

2. The processing system of claim 1, wherein the processing system is configured to iteratively measure:
the at least one first side resistance at a first time interval; and
the at least one second side resistance at a second time interval.

3. The processing system of claim 1 or 2, wherein the processing system is configured to:
compare each first side resistance and each second side resistance to a respective threshold; and
in response to any first side resistance and/or any second side resistance breaching the respective threshold, generating an alert indicator.

4. The processing system of claim 3, wherein generating an alert indicator comprises generating, at a user interface communicatively coupled to the processing system, a user-perceptible output.

5. A defibrillator comprising:
the processing system of any of claims 1 to 4,
the defibrillator paddle, wherein the defibrillator paddle comprises:
the electrode plate for applying an electric shock to a subject, comprising the first side and the second side; and
the plurality of first detection points in electrical contact with the first side of the electrode plate;
the paddle tray configured to receive the defibrillator paddle, wherein the paddle tray comprises the plurality of second detection points configured to, when the defibrillator paddle is received by the paddle tray, come into electrical contact with the second side of the electrode plate.

6. The defibrillator of claim 5, wherein:
the paddle tray comprises a receiving element configured to receive the defibrillator paddle at a first side of the paddle tray; and
each of the plurality of second detection points extends, from a second side of the paddle tray, opposite to the first side of the paddle tray, through a respective hole in the receiving element to the first side of the paddle tray.

7. The defibrillator of any one of claims 5 or 6, wherein each of the plurality of second detection points is a resilient protrusion.

8. The defibrillator of any one of claims 5 to 7, wherein each of the plurality of first detection points is biased, by a respective biasing member, against the first side of the electrode plate.

9. The defibrillator of any one of claims 5 to 8, further comprising:
an electrical shock system configured to, responsive to an activation indicator and only when the electrical shock system is turned on, provide an electric shock using the defibrillator paddle,
wherein the processing system is configured to measure the at least one first side resistance and the at least one of second side resistance, respectively, when the electrical shock system is turned on.

10. The defibrillator of any one of claims 5 to 9, wherein the number of first detection points is different from the number of second detection points.

11. The defibrillator of any one of claims 5 to 10, wherein:
each of the plurality of first detection points is no less than 2 cm away from any other of the plurality of first detection points; and
each of the plurality of second detection points is no less than 2 cm away from any other of the plurality of second detection points.

12. The defibrillator of any one of claims 5 to 11, wherein:
each of the plurality of first detection points electrically contacts a respective first contact point of the first side of the electrode plate; and
each of the plurality of second detection points comes into electrical contact with a respective second contact point of the second side of the electrode plate when the defibrillator paddle is received by the paddle tray.

13. The defibrillator of any one of claims 5 to 12, wherein:
each of the plurality of first detection points is arranged to contact a periphery of the first side of the electrode plate; and
each of the plurality of second detection points is arranged to come into contact with a periphery of the second side of the electrode plate when the defibrillator paddle is received by the paddle tray.

14. A method for measuring resistances of an electrode plate of a defibrillator paddle, the method comprising:
measuring at least one first side resistance of a first side of the electrode plate using a plurality of first detection points in electrical contact with the first side of the electrical plate; and
measuring at least one second side resistance of a second side of the electrode plate using a plurality of second detection points in electrical contact with the second side of the electrical plate, wherein the plurality of second detection points is formed as part of a paddle tray configured to receive the defibrillator paddle.

15. The method of claim 14, further comprising:
comparing each first side resistance and each second side resistance to a respective threshold; and
in response to any first side resistance and/or any second side resistance breaching the respective threshold, generating an alert indicator.
